# EUROPEAN PATENT SPECIFICATION

(11) **EP 4 712 869 B1**
(45) Date of publication and mention of the grant of the patent: **08.07.2026**
(21) Application number: 24727190.1
(22) Date of filing: 14.05.2024
(51) Int. Cl.: A61B 8/08, G01S 15/89

(54) **ULTRASOUND IMAGING SYSTEM AND METHOD FOR IDENTIFYING OPTIMAL ACOUSTIC WINDOW IN TEMPORAL BONE**
ULTRASCHALLBILDGEBUNGSSYSTEM UND VERFAHREN ZUR IDENTIFIZIERUNG EINES OPTIMALEN AKUSTISCHEN FENSTERS IN EINEM ZEITLICHEN KNOCHEN
SYSTÈME D'IMAGERIE ULTRASONORE ET PROCÉDÉ D'IDENTIFICATION DE FENÊTRE ACOUSTIQUE OPTIMALE DANS UN OS TEMPOREL

(30) Priority: 15.05.2023 US 202363466386 P
(43) Date of publication of application: 25.03.2026
(73) Proprietor: Koninklijke Philips N.V., 5656 AE Eindhoven (NL)
(72) Inventor: SHI, William Tao, 5656 AG Eindhoven (NL); MERAL, Faik Can, 5656 AG Eindhoven (NL); RAJU, Balasundar Iyyavu, 5656 AG Eindhoven (NL); YANEZ MORENO, Nicolas, 5656 AG Eindhoven (NL); YOUNIS, Khaled Salem Abdalleh, 5656 AG Eindhoven (NL); PERRONE, Antonio Luigi, 5656 AG Eindhoven (NL); PREVRHAL, Sven Peter, 5656 AG Eindhoven (NL)
(74) Representative: Philips Intellectual Property & Standards
(86) International application number: PCT/EP2024/063168
(87) International publication number: WO 2024/235938

(56) References cited:
- CN-A- 113 576 531
- US-A1- 2012 165 670
- US-A1- 2014 081 144
- VIGNON F ET AL: "Mapping skull attenuation for optimal probe placement in transcranial ultrasound applications", ULTRASONICS SYMPOSIUM (IUS), 2009 IEEE INTERNATIONAL, IEEE, PISCATAWAY, NJ, USA, 20 September 2009 (2009-09-20), pages 2336 - 2339, XP031654713, ISBN: 978-1-4244-4389-5

## Description

### BACKGROUND

Ultrasound imaging has become indispensable for many medical imaging applications. An ultrasound imaging system typically includes an ultrasound probe and a processing system. The probe may include an array of ultrasound transducer elements configured to emit acoustic waves through a patient's body and to receive echo signals as the acoustic waves are reflected from the tissues, organs and other structures. The timing and strength of the echo signals generally correspond to the depth, size, shape, and mass of the structures in the patient's body, images of which are displayed to a user of the ultrasound imaging system.

Transcranial ultrasound, such as color-coded duplex ultrasonography, is widely used due to its capability of assessing both the intracerebral vascular system and anatomical structures, either bone or parenchymal. Because it is a noninvasive and readily available method, transcranial color-coded duplex imaging may be used as a repeatable bedside tool to identify patients with compromised intracranial hemodynamics, already during the ultra-early phase of acute brain injury, thus providing important prognostic information for the clinician.

Therefore, transcranial ultrasound has been developed into a point-of-care modality in prehospital emergency and in-hospital critical care settings. Although transcranial ultrasound is considered relatively straight forward, it does have certain limitations. For example, it relies on the patient having an acoustic window of thin bone, e.g., the temple bone, that allows ultrasound to penetrate the cranium. In addition, transcranial ultrasound is heavily dependent on the skill of the user (e.g., sonographer), making transcranial ultrasound a challenging modality for use by non-experts.

US 2012/165670 A1 discloses an ultrasound imaging method comprising selection of acoustic windows in the temporal bones of both sides of the skull on the basis of an estimated aberration. To this end, so-called aberration maps, particularly a (signal) phase delay map, a (signal) amplitude loss map, and a (signal) waveform distortion map are used.

### SUMMARY

The invention is defined by the claims.

According to a representative embodiment, a method according to claim 1 is provided.

According to another representative embodiment, an ultrasound imaging system according to claim 12 is provided.

According to another representative embodiment, a non-transitory computer readable according to claim 14 is provided.

### BRIEF DESCRIPTION OF THE DRAWINGS

The representative embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A shows an external image of a subject's head, indicating a general location of an optimal acoustic window with reference to facial landmarks.
FIG. 1B shows a backlit ultrasound image of a half skull of the subject's head, indicating the general location of the optimal acoustic window with reference to facial landmarks.
FIG. 2 is a simplified block diagram of an ultrasound imaging system for identifying an optimal acoustic window in a temporal bone of a subject for performing ultrasound imaging, according to a representative embodiment.
FIG. 3 is a flow diagram of a method for identifying an optimal acoustic window in a temporal bone of a subject for performing ultrasound imaging, according to a representative embodiment.
FIG. 4A shows an illustration of high and regular resolution ultrasound images of a subject's head being imaged by an ultrasound probe on the temporal bone, according to a representative embodiment.
FIG. 4B shows an illustration of multiple overlapping 2D footprints the subject's head corresponding to multiple positions of the ultrasound probe while acquiring high and regular resolution ultrasound images, according to a representative embodiment.
FIG. 4C shows a cross-sectional image of an axial view of the subject's head with shallow and in-depth views, according to a representative embodiment.
FIG. 5 shows an illustration of transmitting regular frequency ultrasound transmissions at two transmit angles, according to a representative embodiment.
FIG. 6A shows an illustration of multiple overlapping 2D footprints the subject's head corresponding to multiple positions of the ultrasound probe while acquiring high and regular resolution ultrasound images, according to a representative embodiment.
FIG. 6B shows an extended high resolution reflectivity map including individual extended high resolution reflectivity maps merged together, according to a representative embodiment.
FIG. 6C shows an extended regular resolution transparency map including individual extended regular resolution transparency maps merged together, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

As used in the specification and appended claims, and in addition to their ordinary meanings, the term "about" and "approximately" mean to with acceptable limits or degree. For example, "approximately 2 MHz" means one of ordinary skill in the art would consider the signal to be 2 MHz within reasonable measure. Also, as used in the specification and appended claims, in addition to its ordinary meaning, the term "substantially" means within acceptable limits or degree. For example, the term "substantially simultaneously" means one of ordinary skill in the art would consider occurrence at the same time.

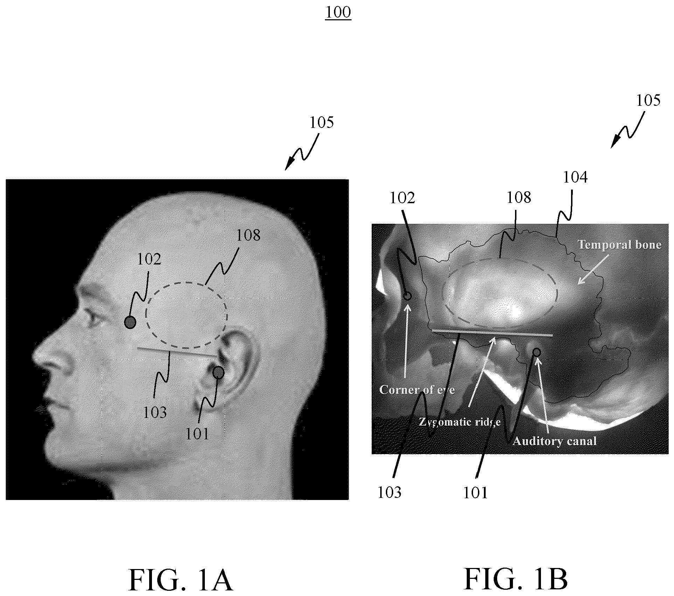

The use of point-of-care transcranial ultrasound is hindered by the difficulty of locating an optimal acoustic window on the temporal bone, especially for non-expert users, and the generally low quality of transcranial ultrasound images otherwise obtained outside the optimal acoustic window. FIGs. 1A and 1B show images of the general location of the optimal acoustic window with reference to facial landmarks, where FIG. 1A shows an external image of a subject's head 105 and FIG. 1B shows a backlit ultrasound image based on a half skull ultrasound image corresponding to the external image.

The subject's head 105 has three facial landmarks, which include external auditory canal 101, lateral canthus of the eye (corner of the eye) 102, and zygomatic ridge 103. Temporal bone 104, outlined in FIG. 1B, encompasses all three of these facial landmarks. The most commonly used acoustic window for ultrasound imaging of the brain is transtemporal window 108 located above the zygomatic ridge 103 between the lateral canthus of the eye 102 and the external auditory canal 101. More particularly, the transtemporal window 108 is generally located above and to the front of the external auditory canal 101, directly behind the lateral canthus of the eye 102, and substantially centered over the zygomatic ridge 103 (e.g., (approximately 10 mm above the auditory canal). The bone is thinner at the transtemporal window 108 to accommodate more effective ultrasound transmissions for an ex-vivo half skull (with backlight illuminated). In FIG. 1B, the transtemporal window 108 is indicated by high brightness from the return echo signals. The ultrasound user typically attempts to locate the general vicinity of the transtemporal window 108 using these rules of thumb.

However, it is difficult to find an optimal acoustic window, e.g., within the transtemporal window 108, owing in part to the inherent inaccuracies of attempting to manually identify the precise region. In addition, attempting to locate the optimal acoustic window using ultrasound imaging is difficult due to significant image degradation from acoustic reverberation at conventional regular frequency ultrasound transmissions because transmitted ultrasound pulses reverberate strongly between the ultrasound probe and temporal bone surfaces. It is also insufficient to define the spatial position of an optimal acoustic window at the conventional regular frequency ultrasound transmissions, which is especially difficult for advanced transcranial imaging techniques, such as positive apex imaging, according to which the apex of an imaging sector is positioned in the center of an acoustic window so that all ultrasound beams travel through the same acoustic window. An optical acoustic window is needed for positive apex imaging.

Generally, according to various embodiments, regular frequency ultrasound transmissions (e.g., about 1.6 MHz to about 3.2 MHz) through an acoustic window are used to acquire individual regular resolution ultrasound images for transcranial imaging. The regular frequency (harmonic) ultrasound transmissions enable sufficient image penetration of the cranium through the temporal bone to obtain a regular resolution transparency map. However, nearfield images of the temporal bone in the acoustic window itself suffer from both low resolution and severe reverberation. Therefore, high frequency ultrasound transmissions (e.g., about 3.6 MHz to about 7.2 MHz) are used to acquire individual high resolution ultrasound images of bone features of the cranium directly beneath two dimensional (2D) imaging footprints of the ultrasound probe at different locations on the temporal bone surface. The higher frequency enables sufficient image resolution of the temporal bone surface to obtain a high resolution reflectivity map. The high frequency ultrasound images are combined with the corresponding regular frequency ultrasound images, so that the instantaneous ultrasound probe location for acquiring both the high and regular frequency ultrasound images can be determined. That is, the regular resolution transparency map is spatially linked to the high resolution reflectivity map, such that the optimal acoustic window may be identified in the low resolution transparency map by tracking the ultrasound probe in the high resolution reflectivity map.

For example, interleaving the high frequency ultrasound transmissions and the regular frequency ultrasound transmissions enables spatial co-registration between locations in resulting high resolution reflectivity maps (and thus on the surface of the temporal bone) and regular resolution transparency maps. As an ultrasound transducer is translated across the temporal bone surface, individual high and regular resolution ultrasound images are generated in each probe footprint of multiple overlapping probe footprints, responsive to the high and regular frequency ultrasound transmissions. Individual 2D high resolution reflectivity maps of the local bone features over the temporal bone surface beneath the ultrasound probe are constructed from three dimensional (3D) high frequency ultrasound imaging, allowing for laterally registering transducer elements of the ultrasound probe (probe footprint) to the rigid temporal bone outer surface.

As further discussed below, individual regular resolution transparency maps are produced from the regular frequency ultrasound transmissions under each probe footprint of the overlapping probe footprints. Echoes from deep inside the brain from multiple transmit angles may be further correlated to remove near-field reverberations. Co-localization of individual regular resolution transparency maps is matched to the corresponding individual high resolution reflectivity maps. Consequently, the spatial position of the optimal acoustic window may be determined as it is automatically co-registered to the high resolution reflectivity maps. Once the high resolution reflectivity maps and the low resolution transparency maps are constructed with spatial co-registration, the user is able to move the ultrasound probe on the temporal bone toward the optimal acoustic window under guidance of the high resolution reflectivity map.

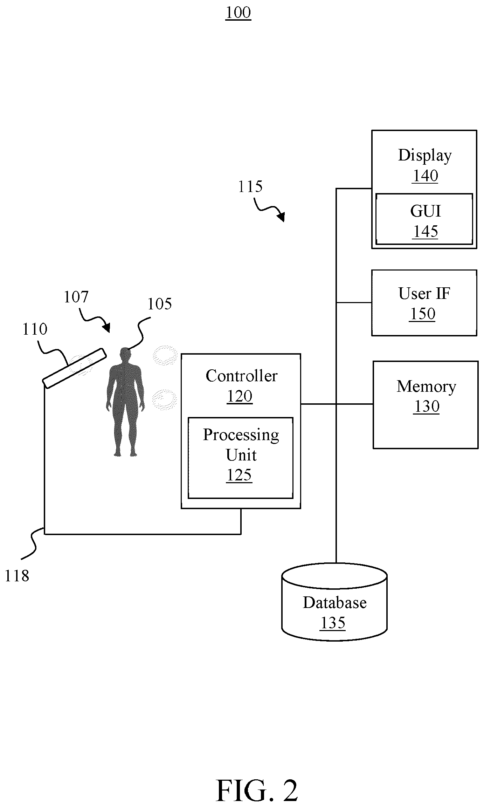

FIG. 2 is a simplified block diagram of an ultrasound imaging system for identifying an optimal acoustic window on a temporal bone of a subject for performing ultrasound imaging, according to a representative embodiment.

Referring to FIG. 2, ultrasound imaging system 100 includes an ultrasound probe 110 and a computer system 115 for controlling imaging of a region of interest (ROI) in the head 105 of subject (patient) 107. The ultrasound probe 110 may include a 2D matrix array of transducer elements, capable of scanning in two or three dimensions, for example, for emitting ultrasound waves into the body of the subject 107 and receiving echo signals in response. The transducer elements may include capacitive micromachined ultrasonic transducers (CMUTs) or piezoelectric transducers formed of materials such as lead zirconate titanate (PZT) or polyvinylidene difluoride (PVDF), for example, although other types of transducer material may be incorporated without departing from the scope of the present teachings. The transducer array elements are coupled to a microbeamformer, often within the ultrasound probe 110, which controls transmission and reception of signals by the transducer elements.

The ultrasound probe 110 is connected to an external controller 120 in the computer system 115 via probe cable 118. Alternatively, all or part of the microbeamformer may be included in the controller 120 for a better thermal control of the probe 110. The controller 120 includes processing unit 125, and is configured to control the ultrasound imaging process, as well as the process for identifying the optimal acoustic window on the temporal bone of the subject 101. The controller 120 includes known elements for performing ultrasound imaging, such as a transmit/receive (T/R) switch configured to switch between transmission and reception modes, e.g., under control of user interface 150 (discussed below), and a main beamformer configured to provide final beamforming following digitization. One of the functions controlled via the controller 120 is the direction in which beams are steered and focused. For example, beams may be steered straight ahead from (orthogonal to) the transducer array of the ultrasound probe 110, or at different angles for a wider field of view. Generally, the transmitting of ultrasound signals and the receiving and processing of echo signals in response is well known, and therefore additional detail in this regard is not included herein.

The computer system 115 receives image data (e.g., B-mode ultrasound images) from the ultrasound probe 110 via the probe cable 118, and processes and stores the imaging data according to representative embodiments described herein. In addition to the controller 120, the computer system 115 includes memory 130, display 140, and the user interface 150. The display 140 may includes a graphical user interface (GUI) 145. In alternative configurations, the computer system 115 may receive the ultrasound images from a database 135, which stores previously acquired ultrasound images of the subject 105.

The memory 130 stores instructions executable by the processing unit 125. When executed, and as described more fully below, the instructions cause the processing unit 125 to perform various processes with regard to identifying an optimal acoustic window in a temporal bone of the subject 101 for performing ultrasound imaging, discussed below. The instructions further allow the user (e.g., sonographer or other clinician) to perform different steps of an exam using the GUI 145 and/or the user interface 150, and to initialize the ultrasound probe 110. In addition, the processing unit 125 may implement additional operations based on executing instructions, such as instructing or otherwise communicating with another element of the computer system 115, including the memory 130 and the display 140, to perform one or more of the processes described herein.

The processing unit 125 is representative of one or more processing devices, and is configured to execute software instructions stored in the memory 130 to perform functions as described in the various embodiments herein. The processing unit 125 may be implemented by a general purpose computer, a central processing unit (CPU), a graphics processing unit (GPU), a computer processor, a microprocessor, a microcontroller, a state machine, programmable logic device, field programmable gate arrays (FPGAs), application specific integrated circuits (ASICs), systems on a chip (SOC), or combinations thereof, using any combination of hardware, software, firmware, hard-wired logic circuits, or combinations thereof. Additionally, any processing unit or processor herein may include multiple processors, parallel processors, or both. Multiple processors may be included in, or coupled to, a single device or multiple devices.

The processing unit 125 may include or have access to an AI engine or module, which may be implemented as software that provides artificial intelligence and applies machine learning, such as neural network modeling. The AI engine may reside in any of various components in addition to or other than the processing unit 125, such as the memory 130, an external server, and/or the cloud, for example. When the AI engine is implemented in a cloud, such as at a data center, for example, the AI engine may be connected to the processing unit 125 via the internet using one or more wired and/or wireless connection(s).

The term "processor" as used herein encompasses an electronic component able to execute a program or machine executable instruction. References to "a processor" should be interpreted to include one processor or more than one processor or processing core, as in a multicore processor. A processor may also refer to a collection of processors within a single computer system or distributed among multiple computer systems, such as in a cloud-based or other multi-site application. Modules have software instructions to carry out the various functions using one or multiple processors that may be within the same computing device or which may be distributed across multiple computing devices.

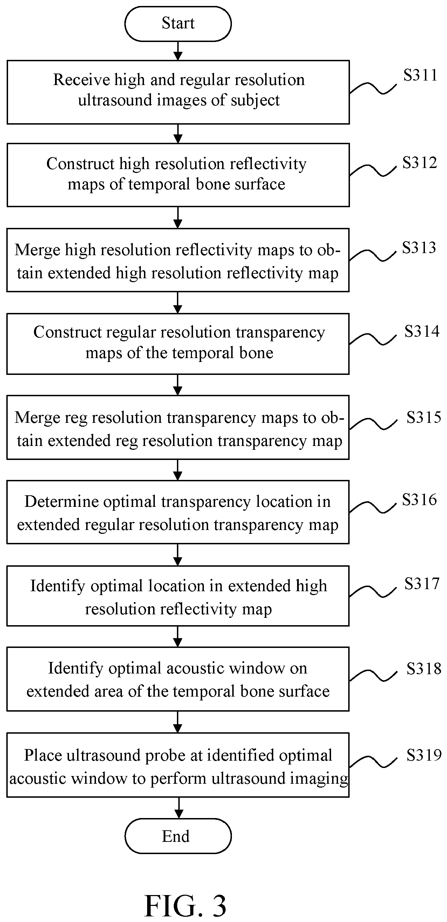

The memory 130 may include a main memory and/or a static memory, where such memories may communicate with each other and the processing unit 125 via one or more buses. The memory 130 stores instructions used to implement some or all aspects of methods and processes described herein. When executed, the instructions cause the processing unit 125 to implement one or more processes for identifying an optimal acoustic window on a temporal bone of a subject for performing ultrasound imaging described below with reference to FIG. 3, for example, as well as to control performance of the ultrasound imaging. The memory 130 may be implemented by any number, type and combination of random access memory (RAM) and read-only memory (ROM), for example, and may store various types of information, such as software algorithms, which serves as instructions, which when executed by the processing unit 125 cause the processing unit 125 to perform various steps and methods according to the present teachings. Furthermore, updates to the methods and processes described herein may also be provided to the computer system 115 and stored in memory 130. The memory 130 may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art.

Each of the memory 130 and the database 135 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. As discussed above, the memory 130 may store software instructions and/or computer readable code that enable performance of various functions, and the database 135 may store previously acquired ultrasound images of the subject 105. The memory 130 and/or the database 135 may be secure and/or encrypted, or unsecure and/or unencrypted.

"Memory" is an example of computer-readable storage media, and should be interpreted as possibly being multiple memories or databases. The memory or database for instance may be multiple memories or databases local to the computer, and/or distributed amongst multiple computer systems or computing devices, or disposed in the "cloud" according to known components and methods. Examples of computer readable storage media include non-transitory media such as computer memory devices that store information in a format that is readable by a computer or data processing system. More specific examples of non-transitory media include computer disks and non-volatile memories.

The display 140 may be any compatible monitor for displaying at least ultrasound images, such as a computer monitor, a television, a liquid crystal display (LCD), a light emitting diode (LED) display, a flat panel display, a solid-state display, or a cathode ray tube (CRT) display, for example. The display 140 may also provide the GUI 145, discussed above, for displaying and receiving information to and from the user. Although the display 140 is shown as it single display, it is understood that the ultrasound imaging system 100 may included multiple displays, for example, one of which is dedicated to displaying ultrasound images in real-time in response to manipulation of the ultrasound probe 110, without departing from the scope of the present teachings.

The user interface 150 is configured to provide information and data output by the processing unit 125 and/or the memory 130 to the user and/or to receive information, instructions and data input by the user. That is, the user interface 150 enables the user to operate the ultrasound imaging system 100 as described herein, including entering imaging settings, such as mode, frequency, depth and gain, for example. Notably, the user interface 150 enables the processing unit 125 to indicate the affects of the user's control or manipulation of the ultrasound probe 110. The user interface 150 may include one or more of ports, disk drives, wireless antennas, or other types of receiver circuitry. The user interface 150 may further connect one or more interface devices, such as a mouse, a keyboard, a mouse, a trackball, a joystick, a microphone, a video camera, a touchpad, a touchscreen, voice or gesture recognition captured by a microphone or video camera, for example.

All or a portion of the user interface 150 may be implemented by the GUI 145 on a touch screen of the display 140, for example. The user interface 150 includes graphics, such as buttons, fields, slides, and other visual markers operable by the user to initiate various commands for manipulating the displayed image, making measurements and calculations, and the like during the ultrasound examination. The push buttons may be displayed by the GUI 145 on the touch screen, for example.

FIG. 3 is a flow diagram of a method for identifying an optimal acoustic window on a temporal bone of a subject for performing ultrasound imaging, according to a representative embodiment. The method may be implemented by the ultrasound imaging system 100, discussed above, under control of the processing unit 125 executing instructions stored in the memory 130, for example.

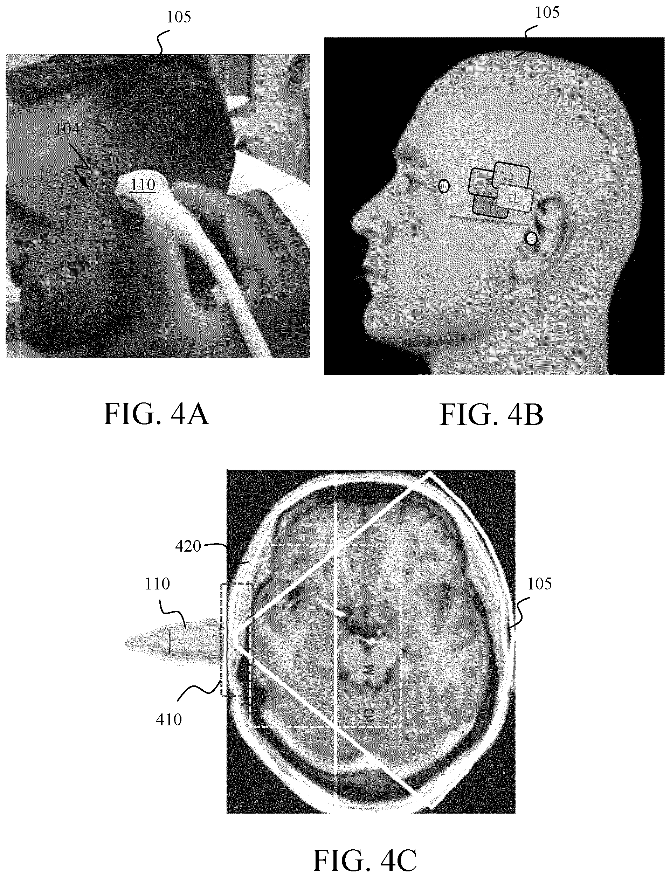

Referring to FIG. 3, high resolution (HRes) ultrasound images and regular resolution (RRes) ultrasound images of the subject are received in block S311, where the high and regular resolution ultrasound images are acquired substantially simultaneously using the ultrasound probe 110. FIG. 4A shows an illustration of high and regular resolution ultrasound images of the subject's head 105 being imaged by the ultrasound probe 110 on temporal bone 104, according to a representative embodiment. The high and regular resolution ultrasound images may be acquired by placing the ultrasound probe 110 over the temporal bone 104 with modest pressure applied. The ultrasound probe 110 is translated gently along the skin contour over an area of the temporal bone 104 that covers the optimal acoustic window. A large number of overlapping individual images from overlapping 2D footprints of the ultrasound probe 110 are generated, as shown for example in FIG. 4B, discussed below.

Generally, the high resolution ultrasound images are acquired using high frequency ultrasound transmissions emitted by the ultrasound probe, and the regular resolution ultrasound images are acquired using regular frequency ultrasound transmissions. For example, the high frequency ultrasound transmissions may be in a high frequency range of about 3.6 to about 7.2 MHz, and the regular frequency ultrasound signals may be in a regular frequency range of about 1.6 to about 3.2 MHz. Due to the lower frequency, the regular frequency ultrasound transmissions have deeper penetration within the subject's cranium through the temporal bone than the high frequency ultrasound transmissions. For example, the regular resolution ultrasound images tend to show parenchymal tissues deep inside the brain, while the high resolution ultrasound images tend to show skin, muscle and the surface of the temporal bone, as shown in FIG. 4C, discussed below. In addition to higher frequency transmissions (resulting in higher resolution in the depth direction), the high resolution ultrasound images are acquired using greater ultrasound beam density than the regular resolution ultrasound images (resulting in higher resolution in the lateral directions), for similar results as far as scanning depth.

As mentioned above, the high and regular resolution ultrasound images are acquired from 2D footprints of the ultrasound probe at corresponding probe positions on an extended area of the temporal bone surface of the subject's temporal bone. The 2D footprints are overlapping, meaning that one or more portions of each 2D footprint covers the same area of the temporal bone surface as at least one other 2D footprint. At least one high resolution ultrasound image and at least one regular resolution ultrasound image are acquired from each of the overlapping 2D footprints. FIG. 4B shows an illustration of multiple overlapping 2D footprints the subject's head 105 corresponding to multiple positions of the ultrasound probe 110 while acquiring the high and regular resolution ultrasound images, according to a representative embodiment. Four overlapping 2D footprints 1, 2, 3 and 4 are shown for purposes of illustration. The four overlapping 2D footprints 1, 2, 3 and 4 are all positioned in the extended area of the temporal bone surface in the expected vicinity of the optimal acoustic window through the temporal bone surface. The high and regular resolution ultrasound images may be acquired substantially simultaneously by interleaving the acquisitions of high resolution ultrasound images and the regular resolution ultrasound images at each of the overlapping 2D footprints 1, 2, 3 and 4 of the ultrasound probe 110. More or fewer overlapping 2D footprints may be incorporated without departing from the scope of the present teachings.

FIG. 4C shows a cross-sectional image of an axial view of the subject's head with shallow and in-depth views, according to a representative embodiment. The ultrasound probe 110 is assumed to be at 2D footprint 3, for example, for acquiring the high and regular resolution ultrasound images substantially simultaneously. As mentioned above, the high frequency ultrasound transmissions are mostly reflected from the temporal bone surface within shallow view 410, and the regular frequency ultrasound transmissions are transmitted beyond the thin temporal bone areas into parenchymal tissues of the brain within in-depth view 420. As shown, both the shallow view 410 and the in-depth view 420 are captured from the same 2D footprint (e.g., 2D footprint 3) of the ultrasound probe 110.

In an embodiment, acquiring the regular resolution ultrasound images includes transmitting the regular frequency ultrasound transmissions at two transmit angles at each of the probe positions over the temporal bone, and correlating echo signals responsive to the regular frequency ultrasound transmissions at the two transmit angles. This technique removes near-field acoustic reverberations.

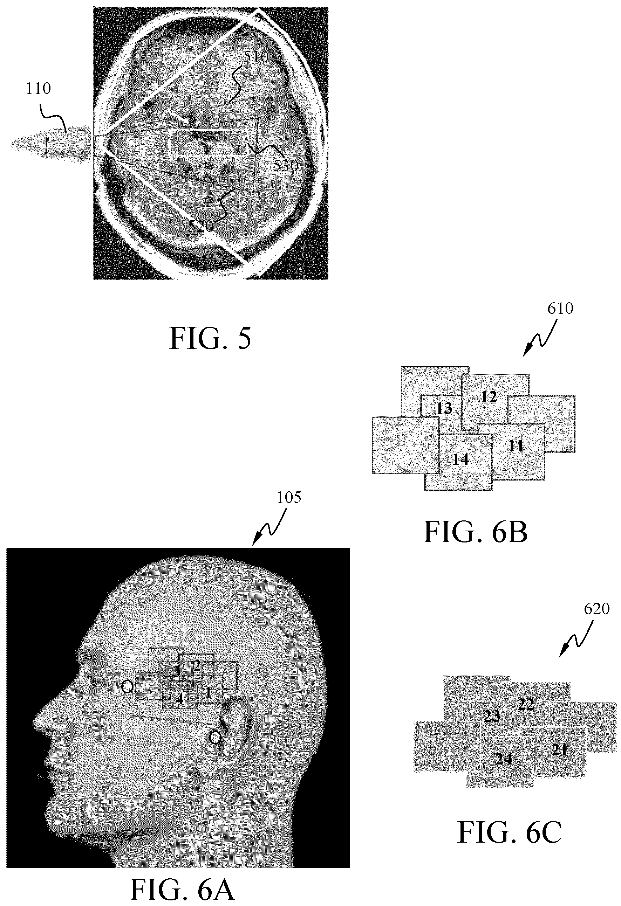

FIG. 5 shows an illustration of transmitting regular frequency ultrasound transmissions at two transmit angles, according to a representative embodiment. Referring to FIG. 5, the regular frequency ultrasound transmissions are conducted at a first transmit angle under a first 2D footprint to acquire regular resolution ultrasound images in a first limited field-of-view 510, and the regular frequency ultrasound transmissions are conducted at a second transmit angle under a second 2D footprint to acquire regular resolution ultrasound images in a second limited field-of-view 520. Echoes (from deep inside the brain) in response to the regular frequency ultrasound transmissions at the first and second transmit angles are correlated to remove the near-field reverberations, as mentioned above. In the depicted embodiment, a deep ROI 530 in an overlapping space of the first and second limited fields-of-view 510 and 520 with limited size may be utilized for faster computations. In this case, the echoes from the regular frequency ultrasound transmissions at the first and second transmit angles are correlated within the ROI 530 to remove the near-field reverberations.

Transmitting the regular frequency ultrasound transmissions at two transmit angles reduces or removes reverberations in the region indicated by the in-depth view 420 in FIG. 4C, although the reverberations originate within the indicated by the shallow view 410. This produces reverberation-free ultrasound images in the in-depth view 420, enabling identification of "true" tissue signals behind the optimal acoustic window without the interference by the reverberations.

In block S312, high resolution reflectivity maps of the temporal bone on the "high density" grid points (i.e., centers of ultrasound beams across the temporal bone outer surface) for the high beam density used in the high frequency imaging are constructed from the high resolution ultrasound images corresponding to the 2D footprints of the ultrasound probe, respectively. Along each ultrasound beam, the reflection from the temporal bone surface is indicated as the maximal amplitude value of received echo signals within a time interval gate of about 2.5 microseconds (open) to about 10 microseconds (close). The time gate range corresponds to a depth range of about 2 mm to about 8 mm (indicated as shallow view 410 in FIG. 4C) within which the temporal bone is located from the probe surface of the ultrasound probe. The reflectivity value along a particular beam is calculated as a ratio of the maximal received signal amplitude for the beam to the transmit signal amplitude. The high resolution reflectivity maps are overlapping where the 2D footprints are overlapping. Generally, the high resolution reflectivity maps include acoustic features distributed over the temporal bone surface, including reflectivity. The outer surface of the skull bone may therefore be used as a 2D reference for spatial coordinates.

In block S313, the high resolution reflectivity maps are merged (spatially registered) to obtain an extended high resolution reflectivity map, which corresponds to the extended area of temporal bone surface. The 2D footprints of the ultrasound probe on the extended area of the temporal bone surface, from which the high resolution reflectivity maps have been acquired, are therefore associated with corresponding locations of the high resolution reflectivity maps in the extended high resolution reflectivity map. Thus, the extended high resolution reflectivity map on the temporal bone surface is automatically registered to the 2D footprints of the ultrasound probe 110, and to corresponding locations of the 2D footprints (probe positions) on the extended area of temporal bone surface itself. The distributive reflectivity pattern (e.g., magnitudes of reflection coefficients within the high frequency band of the high frequency ultrasound transmissions) over the 2D footprint of the ultrasound probe may be utilized as a unique spatial feature of the temporal bone, for example. In an embodiment, the high resolution reflectivity maps may be merged by cross-correlating overlapping portions of the high resolution reflectivity maps, for example, to obtain the extended high resolution reflectivity map.

The extended high resolution reflectivity map provides an extended view of the temporal bone reflectivity. Accordingly, the extended high resolution reflectivity map allows the ultrasound probe 110 to track its footprint position on the temporal bone, e.g., using a cross-correlation algorithm, by matching the current 2D reflectivity pattern (generated corresponding to the current 2D footprint position) to a portion of the extended high resolution reflectivity map.

As an example for the dependency of the local reflectivity on the probe orientation (that is usually slightly varied from the perpendicular position to the temporal bone surface), high frequency ultrasound transmission of a planewave or focused beams with multiple transmit angles (e.g., five angles of [0°, 0°], [5°, 0°], [-5°, 0°], [0°, 5°], [0°, -5°]) along two orthogonal directions on the outer bone surface of the temporal bone may be used for extracting the orientation dependent reflectivity map in depth (within 1-2 mm) at the temporal bone surface. The outer smooth surface of the rigid bone may be employed as the reference for spatial coordinates, where the extended high resolution reflectivity maps along the outer bone surface are spatially registered.

In block S314, regular resolution transparency maps of the temporal bone on the "regular density" grid points (i.e., centers of ultrasound beams across the temporal bone outer surface) for the regular beam density used in the regular frequency imaging are constructed from the regular resolution ultrasound images corresponding to the 2D footprints of the ultrasound probe 110, respectively. Along each ultrasound beam, the transparency of the temporal bone is indicated as the mean amplitude value of received echo signals within a time interval gate of about 10 microseconds (open) to about 100 microseconds (close). The time gate range corresponds to a depth range of about 8 mm to about 80 mm (indicated as the in-depth view 420 in FIG. 4C) within which there is an interior volume beneath the temporal bone, e.g., including the parenchymal tissues of the brain. The transparency value along a particular beam is calculated as a ratio of the mean signal amplitude received along the beam to the transmit signal amplitude. The regular resolution transparency maps are likewise overlapping where the 2D footprints are overlapping. Since the regular resolution ultrasound images are acquired substantially simultaneously with corresponding high resolution ultrasound images, respectively, each regular resolution ultrasound images shares a 2D footprint of the ultrasound probe 110 with a corresponding high resolution ultrasound image.

In block S315, the regular resolution transparency maps are merged (spatially arranged) to obtain an extended regular resolution transparency map of the interior of the subject's head 105 beneath the extended area of the temporal bone surface. The extended regular resolution transparency map is spatially linked (registered) with the extended high resolution reflectivity map, and therefore the 2D footprints of the ultrasound probe on the extended area of temporal bone surface are also associated with corresponding locations of the regular resolution transparency maps in the extended regular resolution transparency map. Accordingly, the extended regular resolution transparency map is co-registered to the same 2D footprints of the ultrasound probe 110 as the extended high resolution reflectively map, and therefore to the corresponding locations of the 2D footprints (probe positions) on the extended area of temporal bone surface. In an embodiment, the regular resolution transparency maps may be merged by averaging the regular resolution transparency maps, for example, to obtain the extended regular resolution transparency map.

In an embodiment, prior to merging, the regular resolution transparency maps may be interpolated into high density transparency maps on the extended area of the temporal bone surface, respectively, so that the density of the transparency maps matches the density of the high resolution reflectivity maps on the same extended area. Transparency map density conversion is performed by interpolating the regular resolution transparency values on the "regular density" grid points (i.e., centers of ultrasound beams across the temporal bone surface) for the regular beam density used in the regular frequency imaging to the "high density" grid points (i.e., centers of ultrasound beams across the temporal bone surface) for the high beam density used in the high frequency imaging. This makes it visually easier to see both the reflectivity and transparency maps with the same high density grid points on the extended area of the temporal bone surface. In this case, the merging in block S315 is performed on the interpolation-matched high density transparency maps to provide an extended high density transparency map.

In block S316, an optimal transparency location is determined in the extended regular resolution transparency map (or corresponding high density transparency map). Determining the optimal transparency location in the extended regular resolution transparency map may include identifying the maximal ultrasound energy/power transmitted through the temporal bone and/or identifying the maximal area within each 2D footprint of the probe positions. The maximal ultrasound energy/power may be determined by summing all transparency values for all ultrasound beams (i.e., the grid points on the temporal surface) associated with the ultrasound probe 110 within each 2D footprint. The maximal area on the temporal surface for each 2D footprint may be identified based on the maximal number of the ultrasound beams associated with the ultrasound probe with transparency values that are above a predetermined threshold, such as a percentile (e.g., 75%) of the maximal transparency value on the extended transparency map. The optimal transparency location may correspond to a translated and/or rotated 2D footprint on the extended high resolution reflectivity map.

In block S317, an optimal location is identified in the extended high resolution reflectivity map, where the optimal location in the extended high resolution reflectivity map corresponds to the optimal transparency location in the extended regular resolution transparency map (or corresponding high density transparency map). The optimal location is identified automatically because the high resolution ultrasound image that shows the optimal location was acquired from the same 2D footprint position of the ultrasound probe 110 as the regular resolution ultrasound image that shows the optimal transparency location. If the grid point interpolation for the high density transparency map has not been performed in block S315, it must be performed in block S317, otherwise block S317 is automatic.

In block S318, the optimal acoustic window is identified in the extended area of the temporal bone surface as a 2D footprint from among the multiple 2D footprints on the extended area of the temporal bone surface that corresponds to the optimal location in the extended high resolution reflectivity map. The optimal acoustic window therefore also corresponds to the optimal transparency location in the extended regular resolution transparency map (or corresponding high density transparency map).

In block S319, the ultrasound probe 110 is placed (translated and/or rotated) to match the identified optimal acoustic window on the extended area of the temporal bone surface to perform ultrasound imaging through the optimal acoustic window. Ultrasound images acquired by the ultrasound probe 110 through the optimal acoustic window may be displayed on the display 140.

FIG. 6A shows an illustration of multiple overlapping 2D footprints the subject's head 105 corresponding to multiple positions of the ultrasound probe while acquiring high and regular resolution ultrasound images, according to a representative embodiment. In particular, FIG. 6A shows the same overlapping 2D footprints 1, 2, 3 and 4 as shown in FIG. 4B, along with three additional overlapping 2D footprints, for purposes of illustration.

FIG. 6B shows an extended high resolution reflectivity map including individual extended high resolution reflectivity maps merged together, according to a representative embodiment. In particular, FIG. 6B shows extended high resolution reflectivity map 610 including merged high resolution reflectivity maps 11, 12, 13 and 14, which respectively correspond to (are co-registered with) the 2D footprints 1, 2, 3 and 4. Similarly, FIG. 6C shows an extended regular resolution transparency map including individual extended regular resolution transparency maps merged together, according to a representative embodiment. In particular, FIG. 6C shows extended regular resolution transparency map 620 including merged regular resolution transparency maps 21, 22, 23 and 24, which respectively correspond to (are co-registered with) the merged high resolution reflectivity maps 11, 12, 13 and 14 and the 2D footprints 1, 2, 3 and 4.

## Claims

1. A method of identifying an optimal acoustic window in a temporal bone of a subject for performing ultrasound imaging, the method comprising:
acquiring high resolution ultrasound images and regular resolution ultrasound images substantially simultaneously from a plurality of 2D footprints of an ultrasound probe corresponding to a plurality of probe positions on an extended area of a temporal bone surface of the temporal bone (S311), wherein the 2D footprints are overlapping;
constructing high resolution reflectivity maps of the temporal bone surface from the high resolution ultrasound images corresponding to the plurality of 2D footprints, respectively (S312), wherein the high resolution reflectivity maps are overlapping where the plurality of 2D footprints are overlapping;
merging the high resolution reflectivity maps to obtain an extended high resolution reflectivity map, wherein the plurality of 2D footprints of the ultrasound probe on the extended area of temporal bone surface are associated with corresponding locations of the high resolution reflectivity maps in the extended high resolution reflectivity map (S313);
constructing regular resolution transparency maps of the temporal bone from the regular resolution ultrasound images corresponding to the plurality of 2D footprints, respectively (S314);
merging the regular resolution transparency maps to obtain an extended regular resolution transparency map (S315), wherein the extended regular resolution transparency map is spatially linked with the high resolution extended reflectivity map such that the plurality of 2D footprints of the ultrasound probe on the extended area of temporal bone surface are further associated with corresponding locations of the regular resolution transparency maps in the extended regular resolution transparency map;
determining an optimal transparency location in the extended regular resolution transparency map (S316);
identifying an optimal location in the extended high resolution reflectivity map corresponding to the optimal transparency location in the extended regular resolution transparency map (S317); and
identifying the optimal acoustic window in the extended area of the temporal bone surface as a 2D footprint of the plurality of 2D footprints corresponding to the optimal location in the extended high resolution reflectivity map for placement of the ultrasound probe to perform the ultrasound imaging (S318).

2. The method of claim 1, further comprising:
placing the ultrasound probe at the identified optimal transparency footprint on the extended high resolution reflectivity map to perform ultrasound imaging through the optimal acoustic window.

3. The method of any one of the preceding claims, wherein the high resolution reflectivity maps are merged by cross-correlating overlapping portions of the high resolution reflectivity maps to obtain the extended high resolution reflectivity map.

4. The method of any one of the preceding claims, wherein the regular resolution transparency maps are merged by averaging the regular resolution transparency maps to obtain the extended regular resolution transparency map.

5. The method of any one of the preceding claims, wherein determining the optimal transparency location in the extended regular resolution transparency map comprises at least one of:
identifying a maximal ultrasound energy/power transmitted through the temporal bone by summing all transparency values for all ultrasound beams associated with the ultrasound probe within each 2D footprint, or
identifying a maximal area within each 2D footprint based on a maximal number of ultrasound beams associated with the ultrasound probe with transparence values above a predetermined threshold.

6. The method of claim 5, wherein the optimal transparency location corresponds to a translated and/or rotated 2D footprint on the extended high resolution reflectivity map.

7. The method of any one of the preceding claims, wherein acquiring the high resolution ultrasound images and the regular resolution ultrasound images substantially simultaneously comprises interleaving acquisitions of high resolution ultrasound images and regular resolution ultrasound images on each of the 2D footprints of the ultrasound probe.

8. The method of any one of the preceding claims, wherein the high resolution ultrasound images are acquired using high frequency ultrasound signals emitted by the ultrasound probe, and the regular resolution ultrasound images are acquired using regular frequency ultrasound signals emitted by the ultrasound probe.

9. The method of claim 8, wherein the high frequency ultrasound signals are in a high frequency range of about 3.6 to about 7.2 **MHz,** and the regular frequency ultrasound signals are in a regular frequency range of about 1.6 to about 3.2 MHz.

10. The method of claim 8 or 9, wherein acquiring the regular resolution ultrasound images comprises:
transmitting regular frequency ultrasound signals at two transmit angles at each position of the plurality of probe positions; and
correlating echo signals responsive to the regular frequency ultrasound signals at the two transmit angles to remove near-field acoustic reverberations.

11. The method of any one of the preceding claims, wherein the high resolution reflectivity maps include a plurality of acoustic features distributed over the temporal bone surface, including reflectivity.

12. An ultrasound imaging system for identifying an optimal acoustic window in a temporal bone of a subject for performing ultrasound imaging, the system comprising:
an ultrasound probe (110) configured to acquire high resolution ultrasound images and regular resolution ultrasound images substantially simultaneously from a plurality of 2D footprints of the ultrasound probe corresponding to a plurality of probe positions on an extended area of a temporal bone surface of the temporal bone, wherein the 2D footprints are overlapping;
a processing unit (125) in communication with the ultrasound probe; and
a non-transitory memory (130) storing instructions that, when executed by the processing unit, cause the processing unit to carry out the method of any one of claims 1 to 11.

13. The system of claim 12, further comprising:
a display configured to display ultrasound images beneath the temporal bone acquired by the ultrasound probe placed at the optimal acoustic window in the extended area of the temporal bone surface.

14. A non-transitory computer readable medium (130) storing instructions, for identifying an optimal acoustic window in a temporal bone of a subject for performing ultrasound imaging, that when executed by a processing unit (125), cause the processing unit to carry out the method of any one of claims 1 to 11.

## Patentansprüche

1. Verfahren zum Identifizieren eines optimalen Schallfensters in einem Schläfenbein eines Subjekts zur Durchführung von Ultraschallbildgebung, wobei das Verfahren umfasst:
Aufnehmen von Ultraschallbildern mit hoher Auflösung und Ultraschallbildern mit normaler Auflösung im Wesentlichen gleichzeitig von einer Vielzahl von 2D-Fußabdrücken einer Ultraschallsonde, die einer Vielzahl von Sondenpositionen auf einem ausgedehnten Bereich einer Schläfenbeinoberfläche (S311) entsprechen, wobei sich die 2D-Fußabdrücke überlappen;
Aufbauen von Reflektivitätskarten mit hoher Auflösung der Schläfenbeinoberfläche aus den Ultraschallbildern mit hoher Auflösung, die jeweils der Vielzahl von 2D-Fußabdrücken entsprechen (S312), wobei sich die Reflektivitätskarten mit hoher Auflösung dort überlappen, wo sich die Vielzahl von 2D-Fußabdrücken überlappen;
Zusammenführen der Reflektivitätskarten mit hoher Auflösung, um eine ausgedehnte Reflektivitätskarte mit hoher Auflösung zu erhalten, wobei die Vielzahl von 2D-Fußabdrücken der Ultraschallsonde auf dem ausgedehnten Bereich der Schläfenbeinoberfläche mit entsprechenden Stellen der Reflektivitätskarten mit hoher Auflösung in der ausgedehnten Reflektivitätskarte mit hoher Auflösung (S313) verknüpft werden;
Aufbauen von Transparenzkarten des Schläfenbeins mit normaler Auflösung aus den Ultraschallbildern mit normaler Auflösung, die jeweils der Vielzahl von 2D-Fußabdrücken entsprechen (S314);
Zusammenführen der Transparenzkarten mit normaler Auflösung, um eine ausgedehnte Transparenzkarte mit normaler Auflösung (S315) zu erhalten, wobei die ausgedehnte Transparenzkarte mit normaler Auflösung räumlich mit der ausgedehnten Reflektivitätskarte mit hoher Auflösung verknüpft ist, sodass die Vielzahl von 2D-Fußabdrücken der Ultraschallsonde auf dem ausgedehnten Bereich der Schläfenbeinoberfläche weiter mit entsprechenden Stellen der Transparenzkarten mit normaler Auflösung in der ausgedehnten Transparenzkarte mit normaler Auflösung verknüpft werden;
Bestimmen einer optimalen Transparenzstelle in der ausgedehnten Transparenzkarte mit normaler Auflösung (S316);
Identifizieren einer optimalen Stelle in der ausgedehnten Reflektivitätskarte mit hoher Auflösung, die der optimalen Transparenzstelle in der ausgedehnten Transparenzkarte mit normaler Auflösung entspricht (S317); und
Identifizieren des optimalen Schallfensters im ausgedehnten Bereich der Schläfenbeinoberfläche als 2D-Fußabdruck der Vielzahl von 2D-Fußabdrücken, die der optimalen Stelle in der ausgedehnten Reflektivitätskarte mit hoher Auflösung zum Platzieren der Ultraschallsonde entsprechen, um Ultraschallbildgebung (S318) durchzuführen.

2. Verfahren nach Anspruch 1, weiter umfassend:
Platzieren der Ultraschallsonde am identifizierten Fußabdruck mit optimaler Transparenz auf der ausgedehnten Reflektivitätskarte mit hoher Auflösung, um Ultraschallbildgebung durch das optimale Schallfenster durchzuführen.

3. Verfahren nach einem der vorstehenden Ansprüche, wobei die Reflektivitätskarten mit hoher Auflösung durch Kreuzkorrelieren überlappender Abschnitte der Reflektivitätskarten mit hoher Auflösung zusammengeführt werden, um die ausgedehnte Reflektivitätskarte mit hoher Auflösung zu erhalten.

4. Verfahren nach einem der vorstehenden Ansprüche, wobei die Transparenzkarten mit normaler Auflösung durch Mitteln der Transparenzkarten mit normaler Auflösung zusammengeführt werden, um die ausgedehnte Transparenzkarte mit normaler Auflösung zu erhalten.

5. Verfahren nach einem der vorstehenden Ansprüche, wobei Bestimmen der optimalen Transparenzstelle in der ausgedehnten Transparenzkarte mit normaler Auflösung mindestens eines umfasst von:
Identifizieren einer maximalen Ultraschallenergie/-leistung, die durch das Schläfenbein übertragen wird, durch Summieren aller Transparenzwerte aller Ultraschallstrahlen, die mit der Ultraschallsonde innerhalb jedes 2D-Fußabdrucks verknüpft sind, oder
Identifizieren eines maximalen Bereichs innerhalb jedes 2D-Fußabdrucks basierend auf einer maximalen Anzahl an Ultraschallstrahlen, die mit der Ultraschallsonde verknüpft sind, mit Transparenzwerten über einem vorbestimmten Schwellenwert.

6. Verfahren nach Anspruch 5, wobei die optimale Transparenzstelle einem verschobenen und/oder gedrehten 2D-Fußabdruck auf der ausgedehnten Reflektivitätskarte mit hoher Auflösung entspricht.

7. Verfahren nach einem der vorstehenden Ansprüche, wobei im Wesentlichen gleichzeitiges Aufnehmen der Ultraschallbilder mit hoher Auflösung und der Ultraschallbilder mit normaler Auflösung Verschachteln von Aufnahmen von Ultraschallbildern mit hoher Auflösung und Ultraschallbildern mit normaler Auflösung auf jedem der 2D-Fußabdrücke der Ultraschallsonde umfasst.

8. Verfahren nach einem der vorstehenden Ansprüche, wobei die Ultraschallbilder mit hoher Auflösung unter Verwendung von Ultraschallsignalen mit hoher Frequenz, die von der Ultraschallsonde ausgesendet werden, aufgenommen werden, und die Ultraschallbilder mit normaler Auflösung unter Verwendung von Ultraschallsignalen mit normaler Frequenz, die von der Ultraschallsonde ausgesendet werden, aufgenommen werden.

9. Verfahren nach Anspruch 8, wobei die Ultraschallsignale mit hoher Frequenz in einem Hochfrequenzbereich von etwa 3,6 bis etwa 7,2 MHz liegen und die Ultraschallsignale mit normaler Frequenz in einem Normalfrequenzbereich von etwa 1,6 bis etwa 3,2 MHz liegen.

10. Verfahren nach Anspruch 8 oder 9, wobei Aufnehmen der Ultraschallbilder mit normaler Auflösung umfasst:
Übertragen von Ultraschallsignalen mit normaler Frequenz in zwei Übertragungswinkeln an jeder Position der Vielzahl von Sondenpositionen; und
Korrelieren von Echosignalen als Reaktion auf die Ultraschallsignale mit normaler Frequenz in den beiden Übertragungswinkeln, um nahegelegene Schallnachhalle zu beseitigen.

11. Verfahren nach einem der vorstehenden Ansprüche, wobei die Reflektivitätskarten mit hoher Auflösung eine Vielzahl von Schallmerkmalen beinhalten, die über die Oberfläche des Schläfenbeins verteilt sind, die Reflektivität beinhaltet.

12. Ultraschallbildgebungssystem zum Identifizieren eines optimalen Schallfensters in einem Schläfenbein eines Subjekts zur Durchführung von Ultraschallbildgebung, wobei das System umfasst:
eine Ultraschallsonde (110), die konfiguriert ist, um im Wesentlichen gleichzeitig Ultraschallbilder mit hoher Auflösung und Ultraschallbilder mit normaler Auflösung von einer Vielzahl von 2D-Fußabdrücken der Ultraschallsonde aufzunehmen, die einer Vielzahl von Sondenpositionen auf einem ausgedehnten Bereich der Schläfenbeinoberfläche entsprechen, wobei sich die 2D-Fußabdrücke überlappen;
eine Verarbeitungseinheit (125) in Verbindung mit der Ultraschallsonde; und
einen nichtflüchtigen Speicher (130), der Anweisungen speichert, die, wenn sie von der Verarbeitungseinheit ausgeführt werden, die Verarbeitungseinheit veranlassen, das Verfahren nach einem der Ansprüche 1 bis 11 auszuführen.

13. System nach Anspruch 12, weiter umfassend:
eine Anzeige, die konfiguriert ist, um Ultraschallbilder unterhalb des Schläfenbeins anzuzeigen, die mit der Ultraschallsonde aufgenommen wurden, die im optimalen Schallfenster im ausgedehnten Bereich der Schläfenbeinoberfläche platziert ist.

14. Nichtflüchtiges, computerlesbares Medium (130), das Anweisungen zum Identifizieren eines optimalen Schallfensters in einem Schläfenbein eines Subjekts speichert, zum Durchführen von Ultraschallbildgebung, die, wenn sie von einer Verarbeitungseinheit (125) ausgeführt werden, die Verarbeitungseinheit veranlassen, das Verfahren nach einem der Ansprüche 1 bis 11 auszuführen.

## Revendications

1. Procédé d'identification d'une fenêtre acoustique optimale dans l'os temporal d'un sujet pour la réalisation d'une imagerie ultrasonore, le procédé comprenant :
l'acquisition d'images ultrasonores à haute résolution et d'images ultrasonores à résolution régulière sensiblement simultanément, à partir d'une pluralité d'empreintes 2D d'une sonde ultrasonore correspondant à une pluralité de positions de sonde sur une zone étendue de la surface de l'os temporal (S311), dans laquelle les empreintes 2D se chevauchent ;
la construction de cartes de réflectivité à haute résolution de la surface de l'os temporal à partir des images ultrasonores à haute résolution correspondant, respectivement, à la pluralité d'empreintes 2D (S312), dans lesquelles les cartes de réflectivité à haute résolution se chevauchent là où la pluralité d'empreintes 2D se chevauchent;
la fusion des cartes de réflectivité à haute résolution pour obtenir une carte de réflectivité à haute résolution étendue, dans laquelle la pluralité d'empreintes 2D de la sonde ultrasonore sur la zone étendue de la surface de l'os temporal sont associées à des emplacements correspondants des cartes de réflectivité à haute résolution dans la carte de réflectivité à haute résolution étendue (S313) ;
la construction de cartes de transparence à résolution régulière de l'os temporal à partir des images ultrasonores à résolution régulière correspondant, respectivement, à la pluralité d'empreintes 2D (S314) ;
la fusion des cartes de transparence à résolution régulière pour obtenir une carte de transparence à résolution régulière étendue (S315), dans laquelle la carte de transparence à résolution régulière étendue est reliée spatialement à la carte de réflectivité étendue à haute résolution de sorte que la pluralité d'empreintes 2D de la sonde ultrasonore sur la zone étendue de la surface de l'os temporal sont associées en outre aux emplacements correspondants des cartes de transparence à résolution régulière dans la carte de transparence à résolution régulière étendue ;
la détermination d'un emplacement de transparence optimale dans la carte de transparence à résolution régulière étendue (S316) ;
l'identification d'un emplacement optimal dans la carte de réflectivité à haute résolution étendue correspondant à l'emplacement de transparence optimale dans la carte de transparence à résolution régulière étendue (S317) ; et
l'identification de la fenêtre acoustique optimale dans la zone étendue de la surface de l'os temporal comme une empreinte 2D parmi la pluralité d'empreintes 2D correspondant à l'emplacement optimal dans la carte de réflectivité à haute résolution étendue pour le placement de la sonde ultrasonore afin de réaliser l'imagerie ultrasonore (S318).

2. Procédé selon la revendication 1, comprenant en outre :
le placement de la sonde ultrasonore au niveau de l'empreinte de transparence optimale identifiée sur la carte de réflectivité à haute résolution étendue afin de réaliser une imagerie ultrasonore à travers la fenêtre acoustique optimale.

3. Procédé selon l'une quelconque des revendications précédentes, dans lequel les cartes de réflectivité à haute résolution sont fusionnées par mise en corrélation croisée de parties superposées des cartes de réflectivité à haute résolution pour obtenir la carte de réflectivité à haute résolution étendue.

4. Procédé selon l'une quelconque des revendications précédentes, dans lequel les cartes de transparence à résolution régulière sont fusionnées en calculant la moyenne des cartes de transparence à résolution régulière pour obtenir la carte de transparence à résolution régulière étendue.

5. Procédé selon l'une quelconque des revendications précédentes, dans lequel la détermination de l'emplacement de transparence optimale dans la carte de transparence à résolution régulière étendue comprend au moins l'une parmi :
l'identification d'une énergie/puissance ultrasonore maximale transmise à travers l'os temporal en additionnant toutes les valeurs de transparence pour tous les faisceaux ultrasonores associés à la sonde ultrasonore dans chaque empreinte 2D, ou
l'identification d'une zone maximale dans chaque empreinte 2D sur la base d'un nombre maximum de faisceaux ultrasonores associés à la sonde ultrasonore avec des valeurs de transparence supérieures à un seuil prédéterminé.

6. Procédé selon la revendication 5, dans lequel l'emplacement de transparence optimale correspond à une empreinte 2D translatée et/ou pivotée sur la carte de réflectivité à haute résolution étendue.

7. Procédé selon l'une quelconque des revendications précédentes, dans lequel l'acquisition des images ultrasonores à haute résolution et des images ultrasonores à résolution régulière comprend sensiblement simultanément, l'entrelacement des acquisitions d'images ultrasonores à haute résolution et d'images ultrasonores à résolution régulière sur chacune des empreintes 2D de la sonde ultrasonore.

8. Procédé selon l'une quelconque des revendications précédentes, dans lequel les images ultrasonores à haute résolution sont acquises à l'aide de signaux ultrasonores à haute fréquence émis par la sonde ultrasonore, et les images ultrasonores à résolution régulière sont acquises à l'aide de signaux ultrasonores à fréquence régulière émis par la sonde ultrasonore.

9. Procédé selon la revendication 8, dans lequel les signaux ultrasonores à haute fréquence se situent dans une plage de hautes fréquences allant d'environ 3,6 à environ 7,2 MHz, et les signaux ultrasonores à fréquence régulière se situent dans une plage de fréquences régulières allant d'environ 1,6 à environ 3,2 MHz.

10. Procédé selon la revendication 8 ou 9, dans lequel l'acquisition d'images ultrasonores à résolution régulière comprend :
la transmission de signaux ultrasonores à fréquence régulière selon deux angles d'émission à chaque position parmi la pluralité de positions de sonde ; et
la corrélation de signaux d'écho répondant aux signaux ultrasonores à fréquence régulière selon les deux angles d'émission pour supprimer les réverbérations acoustiques en champ proche.

11. Procédé selon l'une quelconque des revendications précédentes, dans lequel les cartes de réflectivité à haute résolution incluent une pluralité de caractéristiques acoustiques réparties sur la surface de l'os temporal, y compris la réflectivité.

12. Système d'imagerie ultrasonore permettant d'identifier une fenêtre acoustique optimale dans l'os temporal d'un sujet pour la réalisation d'une imagerie ultrasonore, le système comprenant :
une sonde ultrasonore (110) configurée pour acquérir des images ultrasonores à haute résolution et des images ultrasonores à résolution régulière sensiblement simultanément, à partir d'une pluralité d'empreintes 2D de la sonde ultrasonore correspondant à une pluralité de positions de sonde sur une zone étendue de la surface de l'os temporal, dans laquelle les empreintes 2D se chevauchent ;
une unité de traitement (125) en communication avec la sonde ultrasonore ; et
une mémoire non transitoire (130) stockant des instructions qui, lorsqu'elles sont exécutées par l'unité de traitement, amènent l'unité de traitement à mettre en œuvre le procédé selon l'une quelconque des revendications 1 à 11.

13. Système selon la revendication 12, comprenant en outre :
un écran configuré pour afficher les images ultrasonores sous l'os temporal acquises par la sonde ultrasonore placée au niveau de la fenêtre acoustique optimale dans la zone étendue de la surface de l'os temporal.

14. Support non transitoire lisible par ordinateur (130) stockant des instructions, permettant d'identifier une fenêtre acoustique optimale dans un os temporal d'un sujet pour la réalisation d'une imagerie ultrasonore, qui, lorsqu'elles sont exécutées par une unité de traitement (125), amènent l'unité de traitement à mettre en œuvre le procédé selon l'une quelconque des revendications 1 à 11.
